# EUROPEAN PATENT APPLICATION

(11) **EP 3 766 480 A1**
(43) Date of publication of application: **20.01.2021**
(21) Application number: 20184393.5
(22) Date of filing: 14.05.2015
(51) Int. Cl.: A61K 9/00, A61K 9/46, A61K 31/445

(54) **ORALLY DISINTEGRATING TABLET CONTAINING SOLID LIPID PARTICLES AND METHODS FOR THEIR PREPARATION AND USE**

(30) Priority: 10.06.2014 US 201462010384 P
(62) Divisional of application: 15730266.2
(71) Applicant: Capsugel Belgium NV, 2880 Bornem (BE)
(72) Inventor: SIVERT, Aurélien, 67100 Strasbourg (FR); ANDRÈS, Cyrille, 21610 Saint Maurice sur Vingeanne (FR); BENAMEUR, Hassan, 95600 Eaubonne (FR)
(74) Representative: De Clercq & Partners

(57) **Abstract**

The instant disclosure provides embodiments of orally disintegrating tablet compositions that have rapid disintegrability in the oral cavity and comprise solid lipid particles enclosing at least one active ingredient to mask any unpleasant tastes associated with the active ingredient. Also provided is the process of preparing such orally disintegrating tablet pharmaceutical compositions.

## Description

The present application relates to an orally disintegrating dosage form comprising solid lipid particles.

### BACKGROUND

Solid lipid particle formulations have been disclosed in U.S. Patent 6,572,892; U.S. Patent Application Ser. No. 10/550,027, (published as US 2007-0116728 A1) now abandoned; U.S. Patent Application Ser. No. 12/767,248 (published as US 2010-0221298 A1), now abandoned; and U.S. Patent Application Ser. No. 11/722,267 (published as WO 2006070117). The text of the aforementioned patent and patent applications are hereby incorporated by reference in their entirety.

### SUMMARY

The present disclosure generally relates to an orally disintegrating tablet comprising solid lipid particles comprising various active ingredients or active substances, including biologically active ingredients, pharmaceutical ingredients, pigments or other products, wherein the solid lipid particles minimize or eliminate any unpleasant taste of the substances in the oral cavity and aid in stability of the active ingredient. The active ingredients are taste-masked without the requirement for sugars and/or sweeteners.

One advantage of certain embodiments of the instant disclosure is to provide a more pleasant mouthfeel compared to orally disintegrating tablets made with water or other solvent based granulations.

Other advantages of certain embodiments include increased bioavailability and/or increased stability of the active ingredient and the dosage formulation as a whole, compared to liquid and conventionally granulated forms.

It is to be understood that both the foregoing general description and the following detailed description are exemplary and explanatory only and are not restrictive of the claimed subject matter.

### BRIEF DESCRIPTION OF THE DRAWING

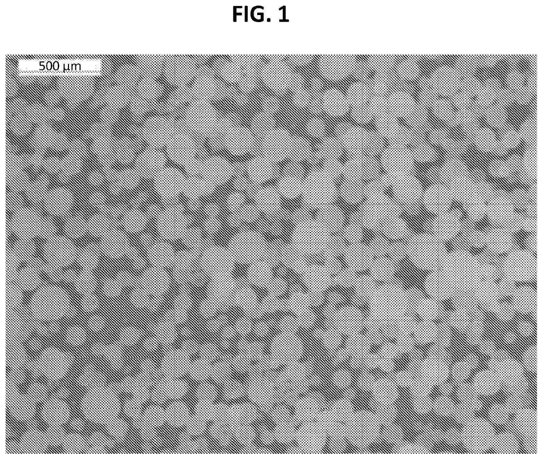

FIG. 1 is a micrograph of solid lipid particles comprising loratadine, according to one embodiment of the presently disclosed pharmaceutical compositions. The mean particle size diameter of the solid lipid particles in this embodiment is smaller than 250 micrometers.

### DETAILED DESCRIPTION

### Definitions

As used herein, reference to an element by the indefinite article "a" or "an" does not exclude the possibility that more than one of the element is present, unless the context clearly requires that there is one and only one of the elements. The indefinite article "a" or "an" thus usually means "at least one." The disclosure of numerical ranges should be understood as referring to each discrete point within the range, inclusive of endpoints, unless otherwise noted. The term "about" as used in the disclosure of numerical ranges indicates that deviation from the stated value is acceptable to the extent that the deviation is the result of measurement variability and/or yields a product of the same or similar properties.

Unless otherwise indicated, all numbers expressing quantities of components, molecular weights, percentages, temperatures, times, and so forth, as used in the specification or claims are to be understood as being modified by the term "about." Accordingly, unless otherwise indicated, implicitly or explicitly, the numerical parameters set forth are approximations that may depend on the desired properties sought and/or limits of detection under standard test conditions/methods. When directly and explicitly distinguishing embodiments from discussed prior art, the embodiment numbers are not approximates unless the word "about" is recited.

Although alternatives are listed in the present disclosure, such lists of alternatives are not to be interpreted as meaning that each alternative is necessarily equivalent to the other.

As used herein, the term "active ingredient," "active substance," "active component," "active pharmaceutical ingredient" and "active agent" is a component that exerts a desired physiological effect on a mammal, including but not limited to humans. In certain embodiments, the "active" as referred to herein may be directed only to humans. Non-limiting examples of active ingredients in certain embodiments include but are not limited to drugs, supplements, dietary supplements, such as vitamins or provitamins A, B, C, D, E, PP and their esters, carotenoids, anti-radical substances, hydroxyacids, antiseptics, molecules acting on pigmentation or inflammation, biological extracts, antioxidants, cells and cell organelles, antibiotics, macrolides, antifungals, itraconazole, ketoconazole, antiparasitics, antimalarials, adsorbents, hormones and derivatives thereof, nicotine, antihistamines, steroid and non-steroid anti-inflammatories, ibuprofen, naproxen, cortisone and derivatives thereof, anti-allergy agents, antihistamines, analgesics, local anesthetics, antivirals, antibodies and molecules acting on the immune system, cytostatics and anticancer agents, hypolipidemics, vasodilators, vasoconstrictors, inhibitors of angiotensin-converting enzyme and phosphodiesterase, fenofibrate and derivatives thereof, statins, nitrate derivatives and anti-anginals, beta-blockers, calcium inhibitors, anti-diuretics and diuretics, bronchodilators, opiates and derivatives thereof, barbiturates, benzodiazepines, molecules acting on the central nervous system, nucleic acids, peptides, anthracenic compounds, paraffin oil, polyethylene glycol, mineral salts, antispasmodics, gastric anti-secretory agents, clay gastric dressings and polyvinylpyrrolidone, aluminum salts, calcium carbonates, magnesium carbonates, starch, derivatives of benzimidazole, and combinations of the foregoing. Orally disintegrating tablets in certain embodiments of the instant disclosure may also comprise a glucuronidation inhibitor, for example, piperine.

Non-limiting examples of active ingredients in certain embodiments include dextromethorphan, fexofenadine, guaifenesin, loratadine, sildenafil, vardenafil, tadafil, Olanzapine, Risperdone, Famotidine, Loperamide, Zolmitriptan, Ondansetron, Cetirizine, Desloratadine, Rizatriptan, Piroxicam, Paracetamol, Phloro-glucinol, Nicergoline, Metopimazine, Dihydroergotamine, Mirtazapine, Clozapine, Zolmitriptan, Prednisolone, Levodopa, Carbidopa, Lamotrigine, Ibuprofen, Oxycodone, Diphenhydramine, Ramosetron, Tramadol, Zolpidem, Fluoxetine, Hyoscyamine,and combinations thereof.

Placebo tablets are also within the scope of certain embodiments. In the case of a placebo tablet, the active substance may be a substance in the excipient of the instant formulation that satisfies the goal of a placebo treatment, which is to objectively impart no specific activity for the condition being treated.

As used herein, the terms "solid lipid particle", "lipidic particle," "lipid particle," "lipid multiparticulate," and "lipidic droplet" should be understood to have substantially the same meaning.

As used herein, the term "flavorant" includes non-toxic, food or pharmaceutical grade excipients that change the flavor of oral pharmaceutical formulations. In some embodiments, a flavorant is not necessary. In other embodiments, a flavorant is included to increase patient compliance, particularly in pediatric patients.

As used herein, the term "substantially free" means the material is not present in the orally disintegrating tablet or the solid lipid particle, as the case may be, in an amount of greater than 0.1 wt%.

As used herein, "w/w %" and "wt%" means by weight as a percentage of the total weight.

### Orally Disintegrating Tablets

In one aspect, the instant disclosure provides orally disintegrating tablet compositions comprising a plurality of solid lipid particles dispersed throughout a mixture of excipients. In certain embodiments the solid lipid particles comprise a single active ingredient and in other embodiments the solid lipid particles comprise more than one active ingredient.

In certain embodiments the orally disintegrating tablets contain in addition to the solid lipid particles a mixture of excipients comprising, consisting essentially of or consisting of a disintegrant, a binder, and/or suitable fillers and/or flavorants. In certain embodiments the orally disintegrating tablets contain in addition to the solid lipid particles a mixture of excipients comprising, consisting essentially of or consisting of a disintegrant, a binder, and optionally suitable fillers and optional flavorants. In certain embodiments the excipient mixture may comprise, consist essentially of or consist of various optional additional components such as diluents, lubricants, anti-sticking aids, and colorants, and mixtures thereof. The orally disintegrating tablets may be coated or non-coated.

In certain embodiments the orally disintegrating tablet includes at least one disintegrant to achieve rapid release of the solid lipid particles when the tablet is placed in an aqueous environment. Non-limiting examples of suitable disintegrants include sodium starch glycolate, crospovidone, polacrilin potassium, a water dispersible cellulose derivative such as microcrystalline cellulose, croscarmellose sodium, hydroxypropyl cellulose, starch, a starch derivative such as sodium carboxymethyl starch, or a mixture thereof. In one embodiment, the disintegrant is a cross-linked polymer such as croscarmellose sodium and/or sodium starch glycolate.

In one embodiment, the disintegrant is selected from the group comprising, consisting essentially of, or consisting of a cross-linked polymer, cellulose, microcrystalline cellulose, methyl cellulose, low-substituted hydroxypropyl methyl cellulose, sodium carboxymethyl cellulose, croscarmellose, hydroxypropyl methyl cellulose, starch, pregelatinized starch, sodium starch glycolate, sodium carboxymethyl starch, and mixtures thereof.

In one embodiment, the disintegrant is selected from the group comprising, consisting essentially of, or consisting of sodium starch glycolate, crospovidone, polacrilin potassium, microcrystalline cellulose, croscarmellose sodium, hydroxypropyl cellulose, starch, sodium carboxymethyl starch, alginic acid, sodium alginate, citric acid, malic acid, tartaric acid, sodium bicarbonate, potassium bicarbonate, calcium carbonate, talc, calcium silicate, silicon dioxide, colloidal silicon dioxide, agar, guar gum, pectin, gellan gum, ion exchange resin, and mixtures thereof.

In one embodiment, the disintegrant is present in the tablet in an amount of from 1 weight percent to 20 weight percent of the total weight of the tablet, or may be from 0.5 to 10 wt%, or may be from 1 to 10 wt%, or may be from 1 to 7wt% of the tablet. In one embodiment the disintegrant is croscarmellose sodium or sodium starch glycolate and is present in 1 to 10 wt% of the tablet.

In certain embodiments the composition also comprises at least one binder that provides structural integrity when the tablet is dry and fast disintegration in the presence of water. An exemplary binder is a polyol, such as mannitol, sorbitol, erythritol, xylitol, and maltitol. Other non-limiting suitable examples of binders include dextrose, sucrose, lactose, maltose, maltodextrin, corn syrup solids, starch, pregelatinized starch, and mixtures thereof, such as sucrose and dextrose, corn syrup solids and mannitol, dextrose and mannitol, starch and mannitol, and maltodextrin and mannitol.

In one embodiment, the binder is preferably mannitol. In another embodiment, the binder may be a mannitol and starch mixture such as Pearlitol® Flash (mannitol/starch ratio 80/20).

In one embodiment, the binder is present in an amount of from 10 weight percent to 60 weight percent of the total weight of the tablet, or may be from 20wt% to 50wt% of the tablet.

The orally disintegrating tablets may in certain embodiments optionally include suitable natural and/or artificial flavorants. Examples include orange flavor, cream flavor, or other flavorants to increase palatablity and patient compliance. Flavorants or flavoring agents may be present in the tablet in an amount from 0.2wt% to 10wt%, preferably from 0.3% to 7wt% of the tablet.

In yet other embodiments, the orally disintegrating tablet is substantially free or completely free from flavoring agents, as the solid lipid particles provide sufficient taste masking for the orally distintegrating tablet to be palatable.

Certain embodiments may include sweeteners to impart a pleasant flavor and greater palatability to the orally disintegrating tablet. Suitable sweeteners for use in the present disclosure include but are not limited to natural sweeteners such as sucrose, dextrose, fructose, invert sugar, mannitol, sorbitol, and the like, and synthetic sweeteners such as saccharin, aspartame, acesulfame potassium, cyclamates, and other commercial artificial sweeteners well-known to those of skill in the art. A preferred sweetener is aspartame or stevia. The sweetener is added in an amount to achieve a desired level of sweetness. The sweetener may be present in an amount from 0.2wt% to 10 wt% of the tablet, and in some embodiments is from 0.5wt% to 5wt%.

In yet another embodiment, the orally disintegrating tablet is substantially free of sweeteners.

Certain embodiments may include a lubricant. The lubricant used may be chosen from pharmaceutically acceptable lubricating agents, including but not limited to magnesium stearate, calcium stearate, sodium stearyl fumarate, stearic acid, micronized polyoxyethylene glycol, microcrystalline cellulose, leucine, sodium benzoate, talc, starches, silica powders, antistatic agents, or combinations thereof.

In one embodiment, the lubricant is magnesium stearate in the range of 0.01 weight percent to 10 weight percent, based on the total weight of the tablet. More preferably, the tablet composition contains 0.5 weight percent to 2 weight percent of magnesium stearate.

Certain embodiments of the orally disintegrating tablet composition may optionally comprise an anti-sticking aid or glidant. Non-limiting examples of anti-sticking aids include talc, calcium carbonate, silica, colloidal silicon dioxide, magnesium trisilicate, starch, tribasic calcium phosphate. In a preferred embodiment, the anti-sticking aid is talc. The quantity of an anti-sticking aid in certain embodiments of the orally disintegrating tablet composition may range from 0.01 weight percent to 10 weight percent, more preferably from 1 weight percent to 5wt%. In another embodiment, the orally disintegrating tablet comprises equal amounts or substantially equal amounts by weight percentage of at least one anti-sticking aid and at least one lubricant.

In certain embodiments, the orally disintegrating tablet composition is substantially free from a surfactant either in the excipient in which the solid lipid particles are dispersed or suspended or within the solid lipid particles.

In certain embodiments, the tablet is substantially free or is completely free of an effervescent system, e.g., lacks citric acid and/or sodium bicarbonate for imparting effervescent disintegrating properties.

In certain embodiments the orally disintegrating tablet may be coated with pharmaceutically acceptable coatings. However, the orally disintegrating tablet does not rely on coating or flavoring to mask any objectionable taste of an active ingredient.

The orally disintegrating tablet may optionally comprise an additive to modify its appearance or rheology. Additives that improve the fluidity of the solid lipid particles may also be included.

### Properties of Orally Disintegrating Tablets

In particular embodiments the orally disintegrating tablet has a physical robustness sufficient to ensure tablet integrity during product life, enabling it to be removed easily from any conventional pharmaceutical packaging without breaking. In certain embodiments, the tablet has physical robustness represented by a breaking force ranging from 15N to 100N. In yet another embodiment, the tablet has a robustness ranging from 20N to 50 N.

The orally disintegrating tablet may be prepared in conventional shapes or geometries. In certain embodiments, the orally disintegrating tablets have a rounded curved shape without sharp edges. While many shapes and sizes are contemplated for the disclosed tablets, the shapes and sizes should be acceptable for oral administration to a mammal, e.g., a human. The tablet diameter in some embodiments is within from 5mm to 20mm, or9mm to 12mm in diameter for administration to humans. In yet another embodiment, the tablet diameter is 10mm.

The tablet has a sufficiently physical robustness, enabling it to be removed easily from any conventional pharmaceutical packaging without breaking. The low friability indicates an improved structural integrity of the orally disintegrating tablet composition against external mechanical forces, allowing the orally disintegrating tablet be packaged using conventional industrial machinery and allowing safe shipping and handling without breaking or eroding. The friability of the orally disintegrating tablet composition in certain embodiments is less than 1%. Preferably, the friability of the tablet composition is 0.5%.

The orally disintegrating tablets disintegrate quickly in aqueous conditions, e.g. saliva or other mucosally secreted solutions, or water, and may be ingested or applied in local or topical treatments, particularly but not exclusively to the mouth or oral cavity. In one embodiment, the orally disintegrating tablet rapidly disintegrates in an oral cavity, i.e., has an initial disintegration time of less than three minutes in water. By disintegration is meant the release of significant portion of the solid lipid particles from the tablet into water; release of the active ingredient from the solid lipid particles may take longer. In certain embodiments, the disintegration of the orally disintegrating tablet is on the order of 10 to 90 seconds, and in other embodiments, the disintegration of the orally disintegrating tablet occurs in an oral cavity in 10 - 60 seconds, or less than 60 seconds.

### Solid Lipid Particles

The solid lipid particles comprise, consist essentially of or consist of an active agent incorporated into a lipid matrix.

The active agent may be any active agent desired to be administered by an orally disintegrating tablet. The solid lipid particles are particularly suitable for administering objectionably-tasting and/or mucosally irritating active ingredients. Suitable active ingredients may have solubility in aqueous solution ranging from practically insoluble, slightly soluble, to freely soluble.

The active agent may be fully solubilized, partially solubilized, or suspended in the lipid matrix. The active agent may be crystalline when suspended in the matrix.

The amount of the active agent present in the solid lipid particles may range from 0.1wt% to 75wt%, or from 0.2wt% to 50wt%, or from 1wt% to 40wt%, or from 2wt% to 30wt%.

In certain embodiments, the instant orally disintegrating composition comprises objectionably-tasting and/or mucosal-irritable active ingredients including but not limited to olanzapine, risperidone, famotidine, loperamide, zolmitriptran, ondansetron, cetirizine, fexofenadine, loratadine; their active metabolites, chemically equivalent forms, any pharmaceutically acceptable salt thereof, and/or mixtures thereof. In yet other embodiments, the orally disintegrating tablet composition containing an active ingredient described herein is useful for treating maladies including but not limited to bipolar disorder, schizophrenia, peptic ulcers, gastric ulcers, duodenal ulcers; diarrhea, migraine; nausea; emesis; and/or allergies.

In one embodiment, the active agent is fexofenadine. As used herein, "fexofenadine" is understood to include the compound and its chemically equivalent forms that substantially retain the pharmacologic properties of fexofenadine, and any pharmaceutically acceptable salt thereof. In certain embodiments, the orally disintegrating composition contains 1mg to 120mg fexofenadine. In certain other embodiments, the orally disintegrating tablet composition contains a generally recommended dose of 30 mg, or may contain a dose of 60mg fexofenadine. In certain embodiments the fexofenadine loading in the solid lipid particles is at least 150mg/g. Other embodiments contain 200mg fexofenadine loaded SLP.

In another embodiment, the active agent is loratadine. Loratadine derivatives such as active metabolites of loratadine, which share the antihistamine properties of loratadine, have been developed. One such metabolite derivative is 8-chloro-6,11-dihydro-11-(4- piperidylidine)-5H-benzo-[5,6]-cyclohepta-[1 ,2-b] pyridine, also known as descarboethoxyloratadine (DCL). As used herein, "loratadine" is understood to include the compound, its active metabolite, and chemically equivalent forms that substantially retain the pharmacologic properties of loratadine, and any pharmaceutically acceptable salt thereof. In certain embodiments, the orally disintegrating composition contains 0.5mg to 20mg loratadine. In other embodiments, the orally disintegrating composition contains a dose of 2.5 mg, 5 mg, or 10 mg loratadine. Certain embodiments include a 100mg loratadine loaded SLP.

The solid lipid particles comprise a lipid matrix. The lipid matrix may comprise, consist essentially of or consist of one or more lipids. The lipids are chosen so that the resulting solid lipid particles are solid at room temperature. The lipid matrix may have an end melting point ranging from 30°C to 85 °C, or may have an end melting point ranging from 35% to 85%, or may have an end melting point of from 35°C to 75°C, or from 37°C to 45°C. By "end melting point" is meant that while the onset of melt may occur at less than the end melting point, at the end melting point temperature the lipid matrix is completely, or in certain embodiments, substantially completely, liquid at 1 atm.

In certain embodiments, the lipid matrix comprises, consists essentially of or consists of natural and/or synthetic oils, fatty acids and their derivatives, glycerides, fatty acid esters, glycolized fatty acid esters, fatty alcohols, sterols and/or waxes.

In certain embodiments, the lipid matrix comprises, consists essentially of or consists of fatty acids such as stearic acid, benzoic acid, citric acid, fumaric acid, lactic acid, and maleic acid.

In certain embodiments, the lipid matrix comprises, consists essentially of or consists of a glyceride. Examples include monoglycerides, diglycerides, triglycerides, etc. with saturated or unsaturated chains having carbon numbers from C6 to C40, e.g. C18 to C24, C8 to C32, C10 to C24, C10 to C18, C12 to C18, etc.), hemisynthetic glycerides or glyceride derivatives with saturated or unsaturated medium to long chain lengths. Exemplary long-chain fatty acid esters include glyceryl monooleate, glyceryl monostearate, glyceryl palmitostearate, mixtures of mono-, di-, and trialkyl glycerides, including mixtures of glyceryl mono-, di-, and tribehenate, glyceryl tristearate, and glyceryl tripalmitate.

In certain embodiments, the lipid matrix comprises, consists essentially of or consists of short to medium chain fatty acid esters, such as isopropyl palmitate, isopropyl myristate, triethyl citrate, lecithin, triacetin, and dibutyl sebacate.

Esters of fatty acids with carbon numbers from C6 to C12 with glycols, e.g. ethylene glycol, propylene glycol, may also be used.

In certain embodiments, the lipid matrix comprises, consists essentially of or consists of a glycolized fatty acid ester, such as polyethylene glycol stearate and polyethylene glycol distearate.

In certain embodiments, the lipid matrix comprises, consists essentially of or consists of a wax. Exemplary waxes include Carnauba wax, Candellila wax, Alfa wax, vegetable waxes, rice wax, hydrogenated jojoba wax or florali absolute waxes, beeswaxes and modified beeswaxes, microcrystalline wax, and paraffin wax.

In certain embodiments, the lipid matrix comprises, consists essentially of or consists of a fatty alcohol. Examples include fatty alcohols with high molecular weight (e.g. cetanol, myristoyl alcohol, stearoyl alcohol).

In certain embodiments, the lipid matrix comprises, consists essentially of or consists of esters of acids and alcohols with high molecular weight, for example, esters of linear and saturated acids with even carbon numbers from C14 to C20, and linear and saturated alcohols with even carbon numbers from C14 to C32.

In certain embodiments, the lipid matrix comprises, consists essentially of or consists of a sterol such as cholesterol and its esters.

In certain embodiments, the lipid matrix comprises, consists essentially of or consists of a vegetable oil such as sunflower oil, olive oil, groundnut oil, and palm oil, as well as hydrogenated vegetable oils, including hydrogenated cottonseed oil.

In certain embodiments, the lipid matrix comprises, consists essentially of or consists of a synthetic oil, such as hydrophobic silicone, cyclomethicones, petroleum waxes or jellies, linear alkanes, lipophilic organic fluorinated oils, perhydrosqualene and/or mixtures thereof. The matrix material may comprise mixtures of materials, such as mixtures of any of the foregoing.

In certain embodiments, the lipid matrix comprises, consists essentially of or consists of a wax, such as those mentioned above, and a non-neutralized fatty acid. The non-neutralized fatty acid may be chosen from fatty acids with linear chains with carbon numbers ranging from C4 to C18, for example such as myristic acid, lauric acid, palmitic acid, or oleic acid and mixtures thereof.

In certain embodiments, the lipid and/or hydrophobic matrix is non-ionizable such that it is not capable of being ionized at neutral physiological pH, i.e., is not able to be ionized at a ph of approximately 7.0. Thus, in some embodiments, the lipid matrix does not include any acid or basic functions, such as organic or mineral bases Certain embodiments of the solid lipid particles are substantially free or are completely free from non-neutralized fatty acids.

Preferred lipid matrix materials include an alkyl-containing glycerol such as a mixture of mono-, di- and triglyceryl behenates (commercially available as COMPRITOL 888, Suppocire ®, a semi-synthetic glyceride base comprising saturated C8-C18 triglyceride fatty acids and Precirol ® (glyceryl distearate) from Gattefosé Corporation of Westwood, N.J.); and hydrogenated cottonseed oil (commercially available as LUBRITAB from Edward Mendell Co. of Patterson, N.Y.).

The lipid matrix may also include, for example for the purposes of adjusting consistency, clays or their oily dispersions, gums of phenylated silicones, starches, and/or fat structuring agents. The lipid matrix may also include a certain number of compounds such as mineral fillers, to modulate density and plasticity. The mineral fillers may be, for example, talc and/or kaolin. In certain embodiments, mineral fillers are not used.

The solid lipid particles may further comprise essential oils, flavorings, pigments, fillers, coloring agents, enzymes and/or coenzymes, preservatives, and/or other active substances or active components.

The amount of lipid matrix present in the solid lipid particles may be from 25% - 90%, or from 25% to 70% or from 1% to 75%, or greater than 25wt% or greater than 50wt%, or may be greater than 60wt% or may be greater than 70wt%, of the total weight of the solid lipid particles.

In certain embodiments the solid lipid particle is substantially free from water.

In certain embodiments, the solid lipid particle is substantially free or is completely free from surfactant, surfactant compounds, and/or surface-active agents.

In certain embodiments the solid lipid particles are substantially insensitive or are completely insensitive to moisture. Thus, in certain embodiments, an active agent is contained in the orally disintegrating tablet disposed in a lipid matrix of one or more hydrophobic compounds and in solid form at ambient temperature and sufficiently free of any surfactants or surfactant compounds, solvent residues, or water that could cause hydrolysis or oxidation reactions of the active ingredient.

Any amount of the solid lipid particles that does not significantly affect the beneficial features of the orally disintegrating tablets, such as friability, robustness, taste, and/or mouthfeel, are within the scope of the disclosure. Generally, the solid lipid particles are present in the finished orally disintegrating tablet of from 0.1wt% to 75wt%, or may be present from 1wt% to 60wt%, or from 5wt% to 50wt%, or from 10wt% to 40wt%.

In some embodiments, solid lipid particles comprising an active ingredient in accordance with the instant disclosure are capable of delayed release of the active ingredient, particularly to avoid release of the active ingredient into the stomach or intestine during ingestion. In some embodiments, solid lipid particles according to the disclosure have one or more additional coatings for delayed release and/or sustained release characteristics.

In certain embodiments, the solid lipid particles are devoid of active ingredient on their surface. Elimination of the active ingredient is obtained, for example, by washing the particles or by a suitable method. Thus, in one embodiment, solid lipid particles in accordance with the present disclosure have no active substance present at their surface. In other embodiments the solid lipid particles are coated with one or more coatings to modify release properties, such as to provide delayed release and/or enteric or colonic release of an active substance.

The solid lipid particles may also include additives to modify the appearance or the rheology. For example, lubricants can be added to the dry powder of particles to improve their fluidity, for example such as talc, starches, silica powders, antistatic agents, and mixtures thereof.

In one embodiment, the solid lipid particles have a substantially spherical shape as shown in FIG. 1. In certain embodiments, the solid lipid particles may have average particle sizes ranging from 0.5 µm to 500 µm in diameter. In one embodiment, the average particle size ranges from 100 µm to 400 µm in diameter, or may range from 120 µm to 250 µm in diameter. In certain embodiments, the active agent in the solid lipid particles has a size ranging from 0.1 µm to 10 µm in average diameter. In certain embodiments, the solid lipid particles have an average particle size of from 0.5 µm to 500 µm or less than 500 µm or less than 250 µm.

### Process for Making

### Solid Lipid Particles

Processes for preparation of solid lipid particles are described in publications WO 1999/65448, WO 2004/084856, U.S. Patent 6,572,892; U.S. Patent Application Ser. No. 10/550,027, (published as US 2007-0116728 A1) now abandoned; U.S. Patent Application Ser. No. 12/767,248 (published as US 2010-0221298 A1), now abandoned; and U.S. Patent Application Ser. No. 11/722,267 (published as WO 2006070117), the disclosures of which are incorporated herein by reference. Other processes for making solid lipid particles are disclosed in US 7,625,507 B2, US 7,951,403, US 7,736,672 B2, and US 7,887,844, the disclosures of which are incorporated herein by reference.

In one embodiment, the solid lipid particles in the instant disclosure are obtained by mixing a lipid phase under moderate heat. For example, wax and oil may be mixed at a temperature corresponding to the melting point of the wax, until the mixture obtained has a melting point lower than the melting point of the wax. The initial ratio of the wax to the oil can be modulated so that the melting point of the end mixture is lower than the degradation temperature of the most heat-sensitive component to be incorporated. In one embodiment, the end mixture is a solid at the temperature of its utilization. For example, the end mixture may have a melting point of 37.5°C. The end mixture is cooled while stirring to a temperature slightly above its melting point, e.g. 2°C or 3°C above its melting point. One or more active ingredients may then be added.

In one embodiment, addition of the active ingredient to the mixture is accomplished such that the ingredient is dispersed throughout - such means include the use of a homogenizer, disperser, or the use of mechanical agitation or stirring. Sonicators or static mixers may be also be used. Other ingredients may be similarly incorporated at the same or different times. In certain preferred embodiments, the active pharmaceutical ingredient, comprising, e.g., fexofenadine and/or other active ingredients, may be subjected to a homogenizing step to form small particles such as nanoparticles of the active pharmaceutical ingredient, prior to its addition to the mixture. The homogenizing step may occur before or after addition of the active pharmaceutical ingredient to the mixture. The mixture is then shaped to give solid lipid particles.

In certain embodiments, shaping is carried out using a gel. The gel may be prepared with a gel-forming/gelifying, shear-thinning, non-surface-active agent or substance, with which the mixture is not miscible. The mixture may be injected into the gel, for example, through an orifice located at the base of a reaction vessel holding the gel, to form a dispersion. Stirring may be continued throughout injection using a blade equipped with an anchor designed to disperse the mixture and a second axial blade equipped with a three-vaned impeller designed to obtain a dispersion having a desired droplet size or a dispersion having a discontinuous phase of a desired characteristic size. The temperature of the gel may be adjusted to be the same as the temperature of the mixture prior to injection. The concentration of the gelifying agent may range, for example, from 0.1 g/l to 30 g/l, e.g. from 0.2 g/l to 20 g/l, or sufficiently high to fix the dispersion. The dispersion may then be cooled below the melting point of the mixture. Solid lipid particles may then be separated from the gel, after which, the solid lipid particles may be washed. The particles may be washed with a mixture of water and ethanol, e.g. a mixture comprising water and up to 25 weight percent of ethanol.

Examples of gelifying agents include carboxyvinyl polymers such as polyacrylic polymers not modified by hydrophobic groups or surfactant groups. Other gelifying agents include carrageenans, thickeners and polysaccharidic gels such as xanthenes, guar and carob gels, alginates, cellulose derivatives, pectins, agar, etc. or mixtures thereof.

After formation, the solid lipid particles may be further processed prior to incorporation into the orally disintegrating tablet. Such further steps may include washing, rinsing, drying, annealing or sieving.

The solid lipid particles and excipients are then mixed together and compressed to form the orally disintegrating tablet. It should be understood that the blending of the solid lipid particles and an excipient can be achieved via different techniques known to those skilled in the art to produce similar results. Once the mixture is thoroughly blended, the blend can be compressed using conventional tableting equipment and standard tablet procedures at a low compaction force.

It should be understood that the embodiments described herein are not limited thereto. Other embodiments of the present disclosure will be apparent to those skilled in the art from consideration of the specification and practice of the disclosed embodiments. The following examples should be considered as exemplary only, with a true scope and spirit of the present disclosure being indicated by the following claims.

### EXAMPLE 1

### Preparation of Lipid Phase

The following materials were melted in a jacketed vessel held at temperature ranging from about 45°C to about 75°C (e.g. about 60°C): a mixture of mono-, di-, and triglycerides having carbon number from C10 to C18 (commercially available as GELUCIRE® 43/01); glycerol dibehenate (commercially available as COMPRITOL® E); lecithin (soy, sunflower), and waxes (e.g. paraffin, beeswax, candelila).

Fexofenadine as the active pharmaceutical ingredient was added at a loading of about 1 weight percent to about 10 weight percent and mixed into the vessel with an agitator at RPM ranging from about 200 RPM to about 600 RPM (e.g. 400 RPM). The resulting mixture of fexofenadine in lipid suspension was maintained at a temperature from about 45 °C to about 75 °C (e.g. 60 °C).

### Preparation of Gel Phase

An appropriate amount of USP water or DI water was weighed and transferred to a jacketed reactor equipped with an agitator. A polyacrylic polymer gelifying agent (commercially available under the trademark CARBOPOL®) was added. The concentration of the gelifying agent was from about 0.1 weight percent to about 0.8 weight percent (e.g. about 0.2 weight percent). The mixture was stirred at from about 200 RPM to about 800 RPM (e.g. about 400 RPM) until the dissolution of the gelifying agent was achieved. The pH of the gel was adjusted with 1N sodium hydroxide to a pH of about 3.5 to about 8.0 (e.g. about 4.5). The gel was then heated to a temperature between about 45°C to about 75°C (e.g. 60°C) under stirring/agitation.

### Flash Injection

While stirring the gel (RPM ranging from about 200 RPM to about 800 RPM, e.g. 500 RPM), the lipid suspension obtained above was transferred via peristaltic pump directly into the gel held in the jacketed reactor. Agitation was terminated upon complete transfer. A dispersion was thus obtained.

### Cooling and Separation

The temperature of the jacketed reactor was adjusted to a temperature of about 2°C to about 10°C (e.g. 5°C). The temperature of the lipid phase of the dispersion was monitored to ascertain hardening. 1N sodium chloride solution was then added to the hardened lipid particles in the gel to break-up the gel. Agitation at about 100 RPM was used to aid gel break-up.

The contents of the jacketed reactor were then transferred to a Buchner funnel equipped with a membrane filter (50 µm to 100 µm pore size) and vacuum capability. Vacuum was applied until the solid lipid particles appeared dry and no more liquid was removed. The particles were washed with water and vacuum filtered again.

The filtered solid lipid particles were transferred to a drying tray and allowed to air dry. Alternatively, vacuum drying was applied until the particles were free-flowing with no apparent moisture may be used. The solid lipid particles were then sieved to obtain a 250 micrometer size distribution and collected.

### EXAMPLE 2

The procedure of Example 1 above was followed as above with additional processing steps including blending the solid lipid particles with anti-sticking aids to reduce tackiness and improve particle flow. Anti-sticking aids such as talc, silica, silicon dioxide, were used at about 0.1 weight percent to about 10 weight percent.

### EXAMPLE 3

The procedure of Example 1 above was followed as above with the exception that the following lipid excipients: Suppocire ®, a semi-synthetic glyceride base comprising saturated C8-C18 triglyceride fatty acids and Precirol ® (glyceryl distearate) from Gattefosse (France) were used in the preparation of the lipid phase. Fexofenadine as the active pharmaceutical ingredient was added. The fexofenadine loading in the resulting solid lipid particles was at least 150mg/g. The solid lipid particles had a particle size smaller than 250 µm.

The composition of fexofenadine-loaded solid lipid particles is summarized in Table 1.

| **TABLE 1** | |
|---|---|
| **Description** | **Composition** |
| SLP w/ fexofenadine | 40 wt.% Suppocire ® A |
| | 40 wt.% Precirol ® ATO55 |
| | 20 wt.% fexofenadine |

### EXAMPLE 4

Orally disintegrating tablets containing the fexofenadine-loaded solid lipid particles from Example 3 above were formed into round curved tablets shape having no sharp edges using conventional low compaction techniques. The tablet diameter was 10 mm. The fexofenadine-loaded solid lipid particles (200mg) accounted for more than 40% of the tablet weight (480mg). The composition of the tablet is summarized in Table 2.

| **TABLE 2** | | |
|---|---|---|
| **Description** | **Composition (mg/tab)** | **Tablet** |
| ODT w/ fexofenadine-loaded SLP | 200mg fexofenadine loaded SLP | 480mg |
| | 10mg Talc | |
| | 200mg Pearlitol® Flash | |
| | 25mg Na Croscarmellose | |
| | 10mg Mg stearate | |
| | 20mg Orange flavor | |
| | 10mg Cream flavor | |
| | 5mg Aspartame | |

The resulting tablets were evaluated for disintegration and breaking strength. The results are presented in Table 3.

| **TABLE 3** | |
|---|---|
| **Properties** | **ODT w/ fexofenadine-loaded SLP** |
| In mouth disintegration time | 76 sec (n=6) |
| Tablet breaking strength | 27 N (n=3) |
| Tablet weight | 480.0 mg |
| Tablet friability | 0.15% |
| Tablet shape | Round curved face |
| Tablet diameter | 10.0 mm |

### EXAMPLE 5

The procedure of Example 1 was followed as above with the exception that the following lipid excipients: Suppocire ® A and Precirol ® from Gatt6foss6 (France) were used in the preparation of the lipid phase. Instead of fexofenadine, loratadine as the active pharmaceutical ingredient was added. The loratadine loading in the resulting solid lipid particles is at least 150mg/g. The solid lipid particles have a particle size smaller than 250 µm.

The composition of Examples 7 is summarized in Table 4.

| **TABLE 4** | |
|---|---|
| **Description** | **Composition** |
| SLP w/ loratadine | 40 wt.% Suppocire ® A |
| | 40 wt.% Precirol ® ATO55 |
| | 20 wt.% loratadine |

### EXAMPLE 6

Orally disintegrating tablets containing the loratadine-loaded solid lipid particles from Example 5 were formed into round curved tablets shape having no sharp edges using conventional low compaction techniques. The tablet diameter was 11 mm. The loratadine-loaded solid lipid particles (100mg) accounted for about 25% of the tablet weight (400mg). Loading of loratadine in the solid lipid particles is 15mg. The composition of the tablet is summarized in Table 6.

| **TABLE 6** | | |
|---|---|---|
| **Description** | **Composition (mg/tab)** | **Dosage** |
| ODT w/ loratadine-loaded SLP | 100mg loratadine loaded SLP | 400mg |
| | 8mg Talc | |
| | 264mg Pearlitol® Flash | |
| | 20mg Na starch glycolate | |
| | 8mg Mg stearate | |
| | 2-5% w/w citric acid | |

### EXAMPLE 7

A lipid blend consisting of a hard-fat and glyceryl palmitostearate (1:1), was melted into which fexofenadine hydrochloride (FXD) was homogeneously dispersed. Using the process of Example 1, spherical solid lipid particles were produced from this heated lipid matrix in an aqueous environment without surfactant/heat, followed by washing with water for optimal taste-masking. Recovered solid lipid particles (<250µm) were compressed with binder and disintegrant to produce orally disintegrating tablets containing 30 mg FXD.

The so obtained solid lipid particles were spherical with a regular smooth surface as confirmed by SEM. The drug loading ratio was >15% and the yield of incorporation >80%. An informal taste panel reported limited compound bitterness in comparison to drug alone, and good mouth-feel using a size fraction of less than 250µm. Dissolution tests in an acidic medium confirmed immediate release of FXD (>60% at 1 hour) with no evidence of compound degradation.

## Claims

1. A pharmaceutical composition, comprising:
An orally disintegrating tablet comprising a disintegrant, a binder, and a plurality of solid lipid particles, said solid lipid particles comprising an active agent and a lipid matrix,
wherein said lipid matrix comprises a lipid selected from the group consisting of: a natural oil, a synthetic oil, a fatty acid and its derivatives, a glyceride, a fatty acid ester, a glycolized fatty acid ester, a fatty alcohol, a sterol, or a wax, and;
wherein said lipid matrix is present in an amount of 50 weight percent or greater of the total weight of said solid lipid particles.

2. The pharmaceutical composition according to claim 1, wherein said binder is present in an amount of from 10 weight percent to 60 weight percent of the total weight of the tablet.

3. The pharmaceutical composition according to claim 1 or 2, wherein said solid particles are present in an amount of from 1 weight percent to 75 weight percent of the total weight of the tablet, preferably of from 5 weight percent to 75 weight percent of the total weight, more preferably of from 10 weight percent to 75 weight percent.

4. The pharmaceutical composition according to any one of claims 1 to 3, wherein said solid lipid particles have an average particle size of 500 micrometers or less.

5. The pharmaceutical composition according to any one of claims 1 to 4, wherein said lipid matrix is present in an amount greater than 70 weight percent of the total weight of the solid lipid particles.

6. The pharmaceutical composition according to any one of claims 1 to 5, wherein said lipid matrix comprises a fatty acid, preferably a fatty acid selected from the group consisting of: stearic acid, benzoic acid, citric acid, fumaric acid, lactic acid, and maleic acid.

7. The pharmaceutical composition according to any one of claims 1 to 6, wherein said lipid matrix comprises a wax, preferably a wax selected from the group consisting of: Carnauba wax, Candellila wax, Alfa wax, vegetable waxes, rice wax, hydrogenated jojoba wax or florali absolute waxes, beeswaxes and modified beeswaxes, microcrystalline wax, and paraffin wax.

8. The pharmaceutical composition according to any one of claims 1 to 7, wherein said lipid matrix comprises a fatty alcohol, preferably cetanol, myristoyl alcohol, or stearoyl alcohol.

9. The pharmaceutical composition according to any one of claims 1 to 8, wherein said lipid matrix comprises a glyceride, preferably a glyceride having carbon numbers from C6 to C40.

10. The pharmaceutical composition according to claim 9, wherein said glyceride is a monoglyceride, diglyceride, or triglyceride.

11. The pharmaceutical composition according to any one of claims 1 to 10, wherein said lipid matrix comprises a long-chain fatty ester, preferably wherein the long-chain fatty acid ester is selected from the group consisting of: glyceryl monooleate, glyceryl monostearate, glyceryl palmitostearate, mixtures of mono-, di-, and trialkyl glycerides.

12. The pharmaceutical composition according to claim 11, wherein said mixture of mono-di, and trialkyl glycerides is a mixture of glyceryl mono-, di-, and tribehenate, glyceryl tristearate, and glyceryl tripalmitate.

13. The pharmaceutical composition according to any one of claims 1 to 12, wherein the orally disintegrating tablet further comprises at least one anti-sticking aid, preferably at least one anti-sticking aid selected from the group consisting of: talc, calcium carbonate, silica, colloidal silicon dioxide, magnesium trisilicate, starch, tribasic calcium phosphate, and combinations thereof.

14. The pharmaceutical composition according to any one of claims 1 to 13, wherein the orally disintegrating tablet further comprises at least one of a flavorant, a sweetener, and combinations thereof, wherein at least one flavorant, sweetener, and combinations thereof are present outside said solid lipid particles.

15. The pharmaceutical composition according to any one of claims 1 to 14, wherein said binder is selected from the group consisting of dextrose, fructose, sucrose, lactose, maltose, mannitol, maltodextrin, colloidal silicon dioxide, corn syrup solids, starch, pregelatinized starch, sorbitol, erythritol, lactitol, fructose, maltitol, trehalose, xylitol, microcrystalline cellulose, gelatin, and combinations thereof.
